# EUROPÄISCHE PATENTSCHRIFT

(11) **EP 0 656 006 B1**
(45) Veröffentlichungstag und Bekanntmachung des Hinweises auf die Patenterteilung: **22.01.1997**
(21) Anmeldenummer: 94908161.6
(22) Anmeldetag: 12.08.1993
(51) Int. Cl.: C07H 15/04

(54) **VERFAHREN ZUR HERSTELLUNG VON ALKYLGLYKOSIDEN**
METHOD OF PREPARING ALKYL GLYCOSIDES
PROCEDE DE FABRICATION D'ALKYLGLUCOSIDES

(30) Priorität: 21.08.1992 DE 4227752
(43) Veröffentlichungstag der Anmeldung: 07.06.1995
(73) Patentinhaber: BASF Aktiengesellschaft, 67063 Ludwigshafen (DE)
(72) Erfinder: WOLF, Gerhard, D-6800 Mannheim 24 (DE); WOLF, Helmut, D-6733 Hassloch (DE)
(86) Internationale Anmeldenummer: EP9302131
(87) Internationale Veröffentlichungsnummer: WO9404544

(56) Entgegenhaltungen:
- EP-A- 0 132 043
- EP-A- 0 415 192
- US-A- 3 598 865

## Beschreibung

Die vorliegende Erfindung betrifft ein Verfahren zur Herstellung von Alkylglykosiden durch Reaktion von Alkoholen mit Monosacchariden oder Verbindungen, die unter den Reaktionsbedingungen Monosaccharide bilden, in Gegenwart von sauren Katalysatoren und anschließende Neutralisation des Reaktionsgemisches mit Basen.

Oberflächenaktive Alkylglykoside, die hauptsächlich auf dem Wasch- und Reinigungsmittelsektor Anwendung finden, sind seit langem bekannt. Sie werden im technischen Maßstab nach zwei unterschiedlichen Verfahren hergestellt, entweder nach der sogenannten Direktsynthese, bei der man die langkettige Alkoholkomponente mit der Zuckerkomponente unter Wasserabspaltung direkt verknüpft oder nach der sogenannten Umacetalisierungsmethode, bei der man zunächst ein kurzkettiges Alkylglykosid herstellt, das dann in einer zweiten Stufe mit langkettigen Alkoholen zum oberflächenaktiven Alkylglykosid umgesetzt wird. Bei beiden Verfahren benötigt man saure Katalysatoren. So ist beispielsweise aus der US-A-3 598 865 bekannt, Schwefelsäure, Salzsäure, Phosphorsäure, phosphorige Säure, Toluolsulfonsäure oder Bortrifluorid als Katalysator einzusetzen. Wie aus der US-A-3 839 318 hervorgeht, kann die direkte Umsetzung von Glykosen und langkettigen Alkoholen auch in Gegenwart von sauren Ionenaustauschern durchgeführt werden. Gemäß der Lehre der EP-B-132 043 verwendet man für die Herstellung von Alkylglykosiden anionische Tenside in der sauren Form als Katalysator. Aus der DE-A-39 27 919 ist bekannt, Sulfobernsteinsäure als Katalysator bei der Herstellung von Alkylglykosiden zu verwenden. Gemäß der Lehre der WO-A-90/07516 verwendet man Dialkylnaphthalinsulfonsäuren als Katalysator bei der Herstellung von Alkylglykosiden. Die obengenannten Katalysatoren haben verschiedene Nachteile. Insbesondere Schwefelsäure und Phosphorsäure führen zur Bildung von Polyglykosen während der Umsetzung der Alkohole mit Monosacchariden. Außerdem erhält man bei Verwendung dieser Katalysatoren stark gefärbte Produkte. Andere Katalysatoren, wie anionische Tenside in der sauren Form, z.B. Dodecylbenzolsulfonsäure, ergeben ebenfalls noch Alkylglykoside, die relativ hohe Farbzahlen aufweisen.

Der vorliegenden Erfindung liegt die Aufgabe zugrunde, ein Verfahren zur Herstellung von Alkylglykosiden zur Verfügung zu stellen, bei dem die Bildung von Polyglykosiden aus den Monosacchariden stark zurückgedrängt ist, und bei dem man Reaktionsprodukte mit einer möglichst niedrigen Iodzahl erhält.

Die Aufgabe wird erfindungsgemäß gelöst mit einem Verfahren zur Herstellung von Alkylglykosiden durch Reaktion von Alkoholen mit Monosacchariden oder Verbindungen, die unter den Reaktionsbedingungen Monosaccharide bilden, in Gegenwart von sauren Katalysatoren und anschließende Neutralisation des Reaktionsgemisches mit Basen, wenn man als Katalysatoren amphotere Tenside in der sauren Form einsetzt. Besonders bevorzugt verwendet man als amphotere Tenside quaternäre Aminoalkylsulfonate, quaternäre Aminoalkylsulfate oder deren Mischungen.

Die amphoteren Tenside werden in der Literatur auch als Sulfobetaine oder Sulfatobetaine bezeichnet. Sie können auch in Form der Alkali- oder Ammoniumsalze als Katalysator eingesetzt werden. In diesem Fall ist es jedoch erforderlich, daraus die freie Säure durch Zugabe einer starken Säure, z.B. Schwefelsäure oder Salzsäure, freizusetzen. Die Freisetzung der Säure kann separat von der Herstellung der Alkylglykoside durchgeführt oder auch im Reaktionsmedium selbst vorgenommen werden.

Die amphoteren Tenside in der sauren Form eignen sich zur Herstellung sämtlicher bisher bekannter Alkylglykoside. Die Alkylglykoside können mit Hilfe der allgemeinen Formel RO(G)ₘ, in der
- G: eine Glykoseeinheit,
- m: eine Zahl von 1 bis 12, vorzugsweise 1 bis 4 und
- R: einen aliphatischen Rest mit 1 bis 30 C-Atomen bedeuten,
charakterisiert werden. Die Glykoseeinheit G kann aus den üblichen Aldosen bzw. Ketosen, z.B. Glukose, Fruktose, Mannose, Galaktose, Talose, Gulose, Allose, Altrose, Idose, Arabinose, Xylose, Lyxose und Ribose bestehen. Auch Glykose-Gemische, Oligosaccharide, z.B. Maltose, Lactose und Palatinose oder Gemische aus Mono- und Oligosacchariden sind möglich. Die Glykosen können sowohl in wasserfreier Form als auch mit Kristallwasser oder auch als wäßriger Sirup eingesetzt werden. Die Verwendung eines wäßrigen Sirups einer Glykose ist aus wirtschaftlichen Gründen bevorzugt. Der Wassergehalt des wäßrigen Sirups der Glykosen beträgt 10 bis 80, vorzugsweise 15 bis 60 Gew.-%. Bevorzugt verwendet man technische Lösungen mit Wassergehalten von 20-40 Gew.-%. Vorzugsweise verwendet man als Glykose wäßrige Glukosesirupe oder Glukose.

Verbindungen, die unter den Reaktionsbedingungen Monosaccharide bilden, sind Oligo- und Polysaccharide. Auch C₁-C₅-Alkylglykoside, die mit C₆-C₃₀-Alkoholen umacetalisiert werden, können als Verbindungen aufgefaßt werden, die unter den Reaktionsbedingungen Monosaccharide bilden.

Die zur Alkylglykosidbildung verwendeten Alkohole können beliebige Kettenlängen haben, d.h. sich von C₁-C₃₀-Alkoholen ableiten. Vorzugsweise werden aliphatische primäre Alkohole eingesetzt. Zur Herstellung von oberflächenaktiven Glykosiden, die als Tensidrohstoffe in wasch - und Reinigungsmitteln eingesetzt werden, setzt man vorzugsweise aliphatische primäre Alkohole mit 6 bis 20, insbesondere mit 8 bis 16 Kohlenstoffatomen mit Monosacchariden oder Verbindungen um, die unter den Reaktionsbedingungen Monosaccharide bilden. Die höheren aliphatischen Alkohole werden beispielsweise aus technischen Fetten hergestellt. Selbstverständlich ist es aber auch möglich, synthetische primäre Alkohole, wie z.B. die sogenannten Oxoalkohole als Reaktionskomponente einzusetzen. Auch Diole können als Alkoholkomponente eingesetzt werden, z.B. Ethylenglykol, Propylenglykole, Butandiole, wie Butandiol-1,4 und Hexandiole, insbesondere Hexandiol-1,6. Als Diole können auch 1,2-und 1,3-Alkandiole der Strukturen 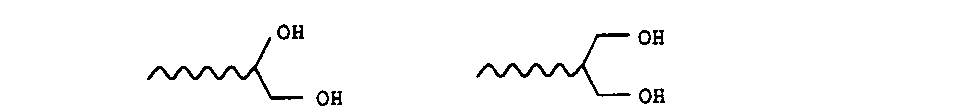 verwendet werden. Bei Verwendung der Diole erhält man Hydroxyalkylglykoside.

Bei den vorzugsweise in Betracht kommenden höheren aliphatischen primären C₈-C₁₈-Alkoholen handelt es sich vorzugsweise um gesättigte und insbesondere um geradkettige Alkohole, die durch Hydrierung von nativen Fettsäuren im technischen Maßstab hergestellt werden. Typische Vertreter der höheren aliphatischen Alkohole sind beispielsweise die Verbindungen n-Dodecylalkohol, n-Tetradecylalkohol, n-Hexadecylalkohol, n-Octadecylalkohol, n-Octylalkohol, Decylalkohol, Undecylalkohol und Tridecylalkohol. Da die Fettalkohole vorzugsweise aus natürlichen Fetten hergestellt werden, liegen üblicherweise Gemische technischer Fettalkohole vor, die auch zur Herstellung der Alkylglykoside eingesetzt werden. Außer den aus natürlichen Fetten gewonnenen Fettalkoholen sind auch verzweigtkettige primäre Alkohole, beispielsweise die sogenannten Oxoalkohole für die Umsetzung geeignet. Typische Oxoalkohole sind z.B. die Verbindungen C₁₂/C₁₃-Alkanol mit ca. 25 % Methylverzweigung, hauptsächlich in der 2-Stellung (Dobanol 23) und der entsprechende C₉-C₁₁-Alkohol (Dobanol 91). Vorzugsweise verwendet man jedoch die aus natürlichen Fetten erhältlichen Alkohole.

Die Reaktion der Alkohole mit den Monosacchariden oder Verbindungen, die unter den Reaktionsbedingungen Monosaccharide bilden, erfolgt erfindungsgemäß in Gegenwart von amphoteren Tensiden in der sauren Form. Man erhält Alkylglykoside der Formel RO(G)ₘ, in der G und R die oben angegebene Bedeutung haben und m eine Zahl von 1 bis 12 ist. Der Index m sollte möglichst klein sein, weil die bei der Umsetzung in untergeordneten Mengen entstehenden Alkylpolyglykoside gegenüber den Alkylmonoglykosiden eine verminderte Waschkraft haben. Der Index m ist daher vorzugsweise eine Zahl von 1,1 bis 1,5. Die bei der Umsetzung entstehenden Alkylglykoside sind Gemische aus Alkylmono- und -oligoglykosiden. Der Index m kann daher auch ungerade Zahlenwerte annehmen. Neben den Alkylglykosiden können auch durch Polymerisation der Glykosen unerwünschte Polyglykosen unterschiedlicher Zusammensetzung gebildet werden. Diese Reaktion ist jedoch bei Verwendung der erfindungsgemäß einzusetzenden amphoteren Tenside in der sauren Form weitgehend zurückgedrängt.

Amphotere Tenside in der sauren Form sind z.B. 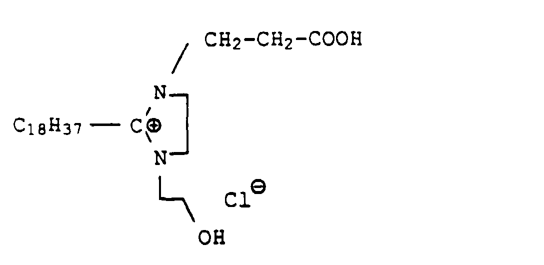 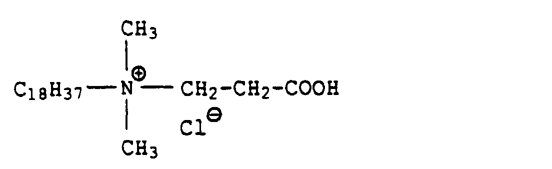 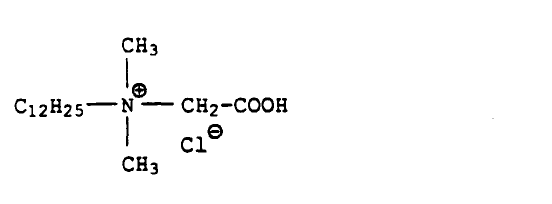 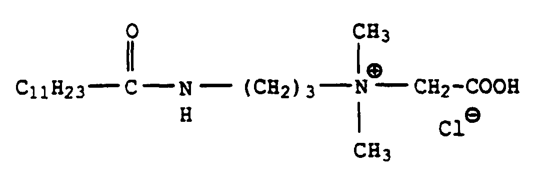 Quaternäre Aminoalkylsulfonate und quaternäre Aminoalkylsulfate in der sauren Form können beispielsweise mit Hilfe der folgenden Formeln charakterisiert werden:

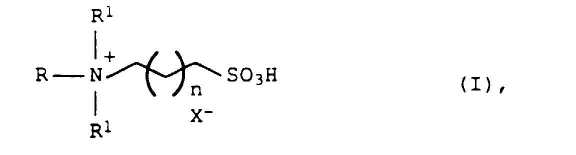

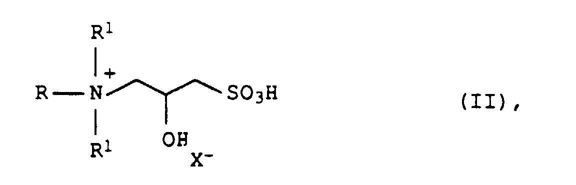

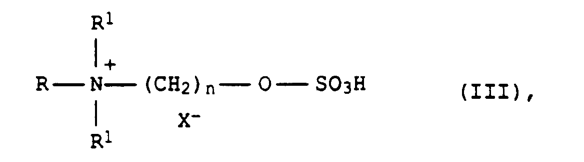

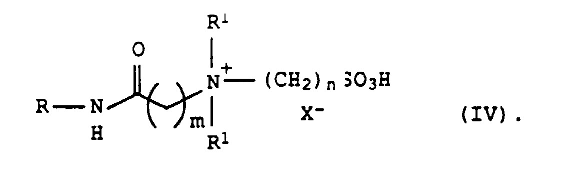

In den Formeln I bis IV bedeutet R - C₈-C₃₀-Alkyl, R¹ - C₁-C₂₀-Alkyl und X ein Anion, vorzugsweise HSO₄⁻, SO₄²⁻ und Cl⁻. Der Index n bedeutet in Formel I 0-10, und in den Formeln III und IV jeweils 2-10. Der Index m in Formel IV steht für 1-6.

Beispiele für die Verbindungen der Formel I sind 3-(N,N-Dimethyl-N-stearyl) -ammonium(propylsulfonsäure) -chlorid und 3-(N,N-Dimethyl(-N-lauryl)-anmoniumlpropylsulfonsäure)-chlorid

Als Verbindungen der Formel II eignen sich beispielsweise 3-(N,N-Dimethyl-N-lauryl)-ammonium-(2-hydroxypropylsulfonsäure)-chlorid und 3- (N,N-Dimethyl-N-stearyl) -ammonium- (2-hydroxypropylsulfonsäure)-chlorid.

Geeignete Verbindungen der Formel III sind z.B. 3-(N,N-Dimethyl-N-lauryl) -ammonium- (propylschwefelsäure) -chlorid oder 3- (N,N-Dimethyl-N-stearylammonium-(propylschwefelsäure)-chlorid.

Ein Beispiel für Verbindungen der Formel IV ist N,N-Dimethyl-N-(N'-stearyl(butyroamid)ammoniumpropylsulfonsäure-chlorid.

Die Verbindungen der oben angegebenen Formeln I bis IV können nicht nur in der sauren Form, sondern auch in der Form der Sulfonsäuresalze als Katalysator bei der Herstellung von Alkylglykosiden eingesetzt werden. Jedoch müssen dann die amphoteren Tenside in der sauren Form durch Zugabe von starken Säuren wie Schwefelsäure oder Salzsäure aus ihren Salzen freigesetzt werden. Die Freisetzung der freien Säuren der amphoteren Tenside aus den Salzen kann auch im Reaktionsmedium durchgeführt werden.

Nach dem erfindungsgemäßen Verfahren werden bevorzugt Alkylglykoside aus Alkoholen mit 6 bis 30 C-Atomen hergestellt. Bezogen auf die bei der Reaktion eingesetzten Monosaccharide verwendet man die amphoteren Tenside in der sauren Form in Mengen von 0,1 bis 5, vorzugsweise 0,1 bis 0,5 Mol-%. Die üblicherweise zum Einsatz gelangenden Katalysatormengen an amphoteren Tensiden in der sauren Form liegen unterhalb der sonst angewandten Katalysatormengen. Bei der direkten Umsetzung von Glukosesirupen mit langkettigen Alkoholen kann auf die Verwendung sonst üblicherweise noch zusätzlich verwendeter Emulgatoren verzichtet werden. Die Alkylglykoside, die unter Verwendung amphoterer Tenside in der sauren Form als Katalysatoren hergestellt werden, weisen eine hellere Farbe auf als mit anderen Katalysatoren hergestellte Alkylglykoside. Außerdem benötigt man zum Bleichen der nach dem erfindungsgemäßen Verfahren erhaltenen Glykoside weniger Bleichmittel als bei Alkylglykosiden, die nach bekannten Verfahren erhalten werden.

Die Prozentangaben in den Beispielen sind Gewichtsprozent.

In den Beispielen wurden folgende Katalysatoren verwendet:
- Katalysator 1:: Lauryldimethylammonium-3-sulfopropylbetain (RALUFON DL), mit Schwefelsäure protoniert
- Katalysator 2:: Cocosfettdimethylammonium-3-sulfopropylbetain (RALUFON DCH), mit Schwefelsäure protoniert
- Katalysator 3:: Stearyldimethylammonium-3-sulfopropylbetain (RALUFON DS), mit Schwefelsäure protoniert
Zum Vergleich mit dem Stand der Technik wurden folgende Katalysatoren getestet:
- Katalysator 4:: Dodecylbenzolsulfonsäure
- Katalysator 5:: p-Toluolsulfonsäure
- Katalysator 6:: Schwefelsäure.

### Beispiel 1a)

In einem 2 l fassenden Mehrhalsrührkolben, der mit Strombrecher, Thermometer, Destillationsaufsatz und einer Dosiereinheit, bestehend aus einer Dosierpumpe, einem Druckhalteventil und einer Düse ausgestattet war, wurden 1033 g eines C₈-C₁₀-Fettalkoholgemisches (Lorol 810 S) vorgelegt und mit 3 g Lauryldimethylammonium-3-sulfopropylbetain (Ralufon DL) und 0,46 g 96 %iger Schwefelsäure versetzt. Die Menge an amphoterem Tensid in der Natriumsalzform betrug 0,5 Mol-%, bezogen auf Glukose. Diese Mischung wurde mit 21 g eines C₈/C₁₀-Alkylglukosid/C₈-C₁₀-Fettalkoholgemisches als Emulgator vermischt. Die Emulgatormischung enthielt ca. 65 % Alkohole und ca. 35 % Glukose. Die Lösung wurde auf eine Temperatur von 115 bis 120°C erhitzt und der Druck auf einen Wert von 50 bis 60 mbar eingestellt. Zu der Vorlage fügte man dann kontinuierlich 485 g eines auf 60°C vorgewärmten. Glukosesirups (65 %ige wäßrige Lösung). Gleichzeitig wurde das mit dem Glukosesirup in das System eingebrachte Wasser abdestilliert. Nach einer Dosierzeit von 4 Stunden hielt man das Reaktionsgemisch noch für 30 Minuten bei einer Temperatur von ca. 120°C. Es resultierte eine leicht trübe, hellgelbe Reaktionslösung, die nach dem Abkühlen mit 50 %iger wäßriger Natronlauge neutralisiert wurde. Anschließend destillierte man den überschüssigen Alkohol ab und verarbeitete den Destillationsrückstand zu einer 60 %igen wäßrigen Lösung, die mit 6 g 30 %iger Wasserstoffperoxidlösung bei 80°C gebleicht wurde. Die Iodfarbzahl der gebleichten Reaktionslösung betrug 5.

### Beispiele 1b) und 1c) sowie Vergleichsbeispiele 1 - 3

Das Beispiel 1a) wurde mit den aus Tabelle 1 ersichtlichen Katalysatoren wiederholt. Die dabei erhaltenen Ergebnisse sind in Tabelle 1 angegeben und zwar der Gehalt an Polyglukose im Reaktionsgemisch, der Wasserstoffperoxidverbrauch für die Bleiche des Reaktionsgemisches und die Farbzahl des gebleichten Reaktionsgemisches.

**Tabelle 1**

| Beispiel 1 | Katalysator Nr. | Polyglucose [%] im Reaktionsgemisch | H₂O₂-Verbrauch [g] | Iodfarbzahl des gebleichten Reaktionsgemisches |
|---|---|---|---|---|
| a) | 1 | 6 | 6,0 | 5 |
| b) | 2 | 8 | 6,0 | 7,5 |
| c) | 3 | 4 | 4,5 | 4 |

| Vergleichsbeispiele | | | | |
|---|---|---|---|---|
| 1 | 4 | 4 | 6,0 | 10 |
| 2 | 5 | 18 | 9,0 | 18,5 |
| 3 | 6 | 25 | 12,0 | 25 |

### Beispiel 2

a) Das Beispiel 1a) wurde mit Katalysator 3 wiederholt.

b) Das Beispiel 1a) wurde mit den Ausnahmen wiederholt, daß man auf den Einsatz des Emulgators verzichtete und den Katalysator 3 anstelle des Katalysators 1 verwendete. Die Ergebnisse sind jeweils Tabelle 2 angegeben.

### Vergleichsbeispiel 4

Das Beispiel 1a) wurde mit der Ausnahme wiederholt, daß man anstelle des dort verwendeten Katalysators jetzt den Katalysator 4 verwendete.

### Vergleichsbeispiel 5

Das Beispiel 1a) wurde mit den Ausnahmen wiederholt, daß man in Abwesenheit des in Beispiel 1a) beschriebenen Emulgators arbeitete und anstelle des dort beschriebenen Katalysators jetzt den Katalysator 4 einsetzte. Die jeweils erhaltenen Ergebnisse sind in Tabelle 2 angegeben.

**Tabelle 2**

| Beispiel 2 | Katalysator Nr. | Polyglucose [%] im Reaktionsgemisch | H₂O₂-Verbrauch [g] | Iodfarbzahl des gebleichten Reaktionsgemisches |
|---|---|---|---|---|
| a | 3 | 4 | 4,5 | 4 |
| b | 6 | 6,0 | 8 | |

| Vergleichsbeispiele | | | | |
|---|---|---|---|---|
| 4 | 5 | 9 | 6,0 | 10 |
| 5 | 4 | ** | 10,0 | 12 |

| | | | | |
|---|---|---|---|---|
| ** Starke Ausfällungen nach der Dosierphase, die zu ca. 80 bis 90 % aus Polyglucose bestand | | | | |

### Beispiel 3

a) Das Beispiel 1a) wurde mit Katalysator 3 wiederholt. Die Katalysatorkonzentration betrug 0,5 Mol-%, bezogen auf Glukose. Die Ergebnisse sind in Tabelle 3 angegeben.

b) Beispiel 1a) wurde wiederholt, indem man anstelle des dort angewendeten Katalysators den Katalysator 3 in einer Menge von 0,1 Mol-%, bezogen auf Glukose einsetzte. Die Ergebnisse sind in Tabelle 3 angegeben.

### Vergleichsbeispiel 6

Das Beispiel 1 wurde mit der einzigen Ausnahme wiederholt, daß man den Katalysator 4 in einer Konzentration von 0,1 Mol-%, bezogen auf Glukose, einsetzte. Die Ergebnisse sind in Tabelle 3 angegeben.

**Tabelle 3**

| Beispiel 3 | Katalysator Nr. | Kat.-Konz. (Mol-% bzgl. Glukose) | Polyglucose [%] im Reaktionsgemisch | Rückstand nach dem Dosieren des Zulaufs |
|---|---|---|---|---|
| a | 3 | 0,5 | 4 | nicht vorh. |
| b | 3 | 0,1 | 4 | nicht vorh. |

| Vergleichsbeispiel | | | | |
|---|---|---|---|---|
| 6 | 5 | 0,1 | 12 | 29 g |

## Patentansprüche

1. Verfahren zur Herstellung von Alkylglykosiden durch Reaktion von Alkoholen mit Monosacchariden oder Verbindungen, die unter den Reaktionsbedingungen Monosaccharide bilden, in Gegenwart von sauren Katalysatoren und anschließende Neutralisation des Reaktionsgemisches mit Basen, dadurch gekennzeichnet, daß man als Katalysatoren amphotere Tenside in der sauren Form einsetzt.

2. Verfahren nach Anspruch 1, dadurch gekennzeichnet, daß man als amphotere Tenside quaternäre Aminoalkylsulfonate, quaternäre Aminoalkylsulfate oder deren Mischungen einsetzt.

3. Verfahren nach Anspruch 1 oder 2, dadurch gekennzeichnet, daß man Alkohole mit 8 bis 30 C-Atomen einsetzt.

4. Verfahren nach Anspruch 1 oder 2, dadurch gekennzeichnet, daß man die amphoteren Tenside in Mengen von 0,1 bis 5 Mol-%, bezogen auf Monosaccharide, einsetzt.

## Claims

1. A process for preparing alkyl glycosides by reacting alcohols with monosaccharides or compounds which, under the reaction conditions, form monosaccharides, in the presence of acid catalysts, and subsequently neutralizing the reaction mixture with bases, wherein amphoteric surfactants in the acid form are employed as catalysts.

2. A process as claimed in claim 1, wherein quaternary aminoalkylsulfonates, quaternary aminoalkyl sulfates or mixtures thereof are employed as amphoteric surfactants.

3. A process as claimed in claim 1 or 2, wherein alcohols with from 8 to 30 carbons are employed.

4. A process as claimed in claim 1 or 2, wherein the amphoteric surfactants are employed in amounts of from 0.1 to 5 mol % based on monosaccharides.

## Revendications

1. Procédé de fabrication d'alkylglucosides par réaction d'alcools avec des monosaccharides ou des composés qui forment des monosaccharides dans les conditions réactionnelles, en présence de catalyseurs acides et neutralisation ultérieure du mélange réactionnel avec des bases, caractérisé en ce que l'on utilise comme catalyseurs des tensio-actifs amphotères dans la forme acide.

2. Procédé selon la revendication 1, caractérisé en ce que l'on utilise comme tensio-actifs amphotères des aminoalkylsulfonates quaternaires, des aminoalkylsulfates quaternaires, ou leur mélange.

3. Procédé selon les revendications 1 ou 2, caractérisé en ce que l'on utilise des alcools ayant 8 à 30 atomes de carbone.

4. Procédé selon les revendications 1 ou 2, caractérisé en ce que l'on utilise les tensio-actifs amphotères en quantité de 0,1 à 5% en moles, par rapport aux monosaccharides.
